# EUROPÄISCHE PATENTSCHRIFT

(11) **EP 2 946 641 B1**
(45) Veröffentlichungstag und Bekanntmachung des Hinweises auf die Patenterteilung: **07.12.2016**
(21) Anmeldenummer: 14703022.5
(22) Anmeldetag: 02.01.2014
(51) Int. Cl.: H05H 1/24, A61L 2/14

(54) **PLASMA-BEHANDLUNGSGERÄT MIT EINER DREHBAR IN EINEM GRIFFGEHÄUSE GELAGERTEN ROLLE**
PLASMA TREATMENT DEVICE COMPRISING A ROLLER MOUNTED ROTATABLY IN A HANDLE HOUSING
DISPOSITIF DE TRAITEMENT AU PLASMA COMPORTANT UN ROULEAU ROTATIF LOGÉ DANS UNE POIGNÉE

(30) Priorität: 15.01.2013 DE 102013000440
(43) Veröffentlichungstag der Anmeldung: 25.11.2015
(73) Patentinhaber: Cinogy GmbH, 37115 Duderstadt (DE)
(72) Erfinder: TRUTWIG, Leonhard, 37115 Duderstadt (DE); STORCK, Karl-Otto, 37115 Duderstadt (DE); WANDKE, Dirk, 37308 Heilbad Heiligenstadt (DE); KOPP, Matthias, 37434 Gieboldehausen (DE); DÄSCHLEIN, Georg, 16348 Wandlitz OT Klosterfelde (DE)
(74) Vertreter: Lins, Edgar
(86) Internationale Anmeldenummer: PCT/DE2014/000001
(87) Internationale Veröffentlichungsnummer: WO 2014/111081

(56) Entgegenhaltungen:
- WO-A1-2009/003613
- WO-A1-2011/076193
- WO-A1-2012/053083
- WO-A2-2006/116252
- WO-A2-2013/156352
- JP-A- 2003 142 415

## Beschreibung

Die Erfindung betrifft ein Plasma-Behandlungsgerät zur Behandlung einer Oberfläche mit einem dielektrisch behinderten Plasmafeld, das zwischen einer mit Hochspannung versorgten Elektrode und der Oberfläche erzeugt wird, wobei die Elektrode mit einem die Elektrode umgebenden Dielektrikum eine drehbar in einem Griffgehäuse gelagerte Rolle bildet, die auf der Oberfläche abrollbar ist.

Die Behandlung von Oberflächen mit einem Plasma ist zu den verschiedensten Zwecken bekannt. So können Kunststoffoberflächen, die sich ohne Vorbehandlung nicht oder nur schwierig beschichten, beispielsweise lackieren, lassen, nach einer Plasmabehandlung wesentlich besser beschichtet werden. Ähnliches gilt für Keratin-Oberflächen, wie beispielsweise Haare, die nach einer Plasmabehandlung wesentlich besser Behandlungs- oder Pflegemittel aufnehmen. Bekannt ist ferner, Oberflächen mit einer Plasmabehandlung zu desinfizieren. Eine Plasmabehandlung ist auch am lebenden Körper möglich, um die Aufnahmefähigkeit der Haut für Pflege- und Heilmittel zu verbessern, insbesondere für die Wundheilung, und beanspruchte Oberflächen zu desinfizieren.

Während für die Plasmabehandlung zur Materialbearbeitung das Plasmafeld unmittelbar zwischen zwei Elektroden generiert werden kann, ist es in vielen Fällen zweckmäßig, eine sogenannte dielektrisch behinderte Plasmaentladung vorzusehen. Bei einer dielektrisch behinderten Plasmaentladung wird Luft oder ein anderes Gas durch ein Hochspannungsfeld ionisiert, der aufgrund der hohen Potentialdifferenz an sich entstehende Stromfluss jedoch durch ein zwischengeschaltetes Dielektrikum unterbunden. Es ist daher bekannt, eine Hochspannungselektrode in ein Dielektrikum einzubetten, um einen Stromüberschlag sicher zu verhindern und das Plasmafeld mit einer Gegenelektrode zu produzieren, wobei die zu behandelnde Oberfläche als Gegenelektrode, die üblicherweise an Masse gelegt wird, dienen kann, wenn das Material der zu behandelnden Oberfläche elektrisch leitend ist. Durch WO 2011/076193 A1 ist eine flächige Anordnung aus einer in ein flexibles Dielektrikum eingebetteten flächigen Elektrode bekannt, die auf eine Oberfläche auflegbar ist und sich aufgrund ihrer Flexibilität an Unebenheiten der Oberfläche in einem gewissen Maß anpassen kann. Die Oberfläche der Elektrodenanordnung ist dabei mit noppenähnlichen Erhöhungen versehen, um beim Auflegen auf die zu behandelnde Oberfläche in den Zwischenräumen zwischen den Erhöhungen Lufträume zu gewährleisten, in denen sich das Plasma ausbilden kann. Eine derartige Anordnung ist nicht universell einsetzbar, da die Größe der Elektrodenanordnung an die Größe der zu behandelnden Fläche angepasst sein muss, um eine sinnvolle rationelle Behandlung der Oberfläche zu ermöglichen.

WO2006/116252 A2 offenbart eine dielektrische Barriere-Entladungs Vorrichtung mit einer Rollenelektrode.

In einer Internet-Veröffentlichung der MBM ScienceBridge GmbH, Göttingen, ist ein sogenannter Plasma-Roller bekannt, der aus einem keramikummantelten Kupferrohr als Elektrode besteht. Die Rolle wird über die zu behandelnde Oberfläche nach Art eines Farbrollers geführt, sodass sie mit der Oberfläche einen linienförmigen Kontakt ausbildet. Das Plasmafeld bildet sich offenbar beiderseits des linienförmigen Kontakts aus. Der Plasmaroller soll zum Desinfizieren von Wänden, Böden oder Einrichtungsgegenständen sowie zur Vorbehandlung dieser Gegenstände für einen Anstrich oder andere Beschichtungen ohne Lösungsmittel und Primer geeignet sein, wenn der Plasmaroller mehrfach über die zu behandelnde Oberfläche geführt wird.

Die vorbekannte Konstruktion des Plasmarollers ist nur für glatte Oberflächen geeignet und weist den Nachteil auf, dass sich in Abhängigkeit von dem Abstand zur Kontaktlinie des Plasmarollers mit der Oberfläche stark abfallende Plasmafelder ausbilden, sodass eine kontrollierte und definierte Plasmabehandlung nicht möglich ist.

Der vorliegenden Erfindung liegt daher die Aufgabe zugrunde, das Anwendungsgebiet für eine als Rolle ausgebildete Elektrodenanordnung für eine Plasmabehandlung zu erweitern und eine besser definierte und kontrollierte Plasmabehandlung zu ermöglichen.

Zur Lösung dieser Aufgabe ist ein Plasma-Behandlungsgerät der eingangs erwähnten Art erfindungsgemäß dadurch gekennzeichnet, dass die Rolle an Unebenheiten der Oberfläche flexibel anpassbar ausgebildet ist und eine Abrollfläche mit Erhöhungen aufweist, zwischen denen sich das Plasmafeld ausbildende Zwischenräume befinden.

Das erfindungsgemäße Plasma-Behandlungsgerät weist somit eine Rolle auf, die sich flexibel an Unebenheiten der Oberfläche anpassen kann. Hierzu ist das Dielektrikum insbesondere aus einem weichelastischen Material und vorzugsweise mit einer Härte Shore A zwischen 30 und 60, gebildet. Geeignete weichelastische Materialien sind thermoplastische Elastomere (TPE). Ein erfindungsgemäßes Plasma-Behandlungsgerät kann somit bereits durch die weichelastische Ausbildung des Dielektrikums an die Unebenheiten der Oberfläche anpassbar sein, auch wenn die Elektrode selbst starr ist. In einer bevorzugten Ausführungsform der Erfindung ist jedoch auch die Elektrode flexibel ausgebildet, besteht somit aus einem flexiblen Material, mit starren Endstücken in dem Griffgehäuse drehbar gelagert ist. Die Lagerung erfolgt vorzugsweise an beiden Enden der Elektrode. Möglich ist aber auch eine einseitige Lagerung der Elektrode, die somit mit einem freien Ende ausgebildet sein kann.

Die Oberfläche der Rolle ist erfindungsgemäß ferner mit Erhöhungen versehen, zwischen denen sich Zwischenräume befinden, in denen sich das Plasmafeld ausbildet. Die Rolle weist dabei zahlreiche Erhöhungen auf, die regelmäßig oder unregelmäßig auf der Oberfläche angeordnet sein können. Die Zwischenräume zwischen den Erhöhungen sind vorzugsweise miteinander verbunden, sodass sich um die Erhöhungen herum ein einheitlicher Gasraum ausbilden kann, wenn die Rolle an der zu behandelnden Oberfläche - ggf. unter Druck - anliegt. Durch die weiche Ausbildung des Dielektrikums und ggf. die Flexibilität der Elektrode bildet sich auch im Kontaktbereich zwischen der Rolle und der zu behandelnden Oberfläche ein relativ breites Plasmafeld aus, das nur an den Auflagepunkten unterbrochen wird, die keine ununterbrochene Linie bilden, sondern entlang einem Kontaktstreifen zwischen der Oberfläche der Rolle und der zu behandelnden Oberfläche nur punktuelle Kontaktflächen bilden, zwischen denen sich ein Plasmafeld in einem Zwischenraum zwischen den Kontaktpunkten ausbilden kann. Da sich beim Abrollen die Lage der Kontaktpunkte ständig ändert, ergibt sich so ein relativ breites Plasmafeld mit einer annähernd homogenen Feldstärke, durch das eine kontrollierte und definierte Plasmabehandlung der Oberfläche möglich ist.

Das erfindungsgemäße Plasma-Behandlungsgerät eignet sich für alle oben erwähnten Anwendungsfelder, für die die bekannten Plasmageräte geeignet sind. Darüber hinaus ermöglicht das erfindungsgemäße Plasma-Behandlungsgerät eine Behandlung von unebenen Oberflächen beliebiger Größe mit einer dennoch besser definierten Plasmabehandlung. Dies ist insbesondere für die Plasmabehandlung der Haut eines lebenden Körpers wichtig, da eine kontrollierte Behandlung, beispielsweise der Gesichtshaut oder der Haut an anderen Körperteilen mit einem erfindungsgemäßen Plasma-Behandlungsgerät mit einer geeignet gewählten Rollengröße ermöglicht wird.

Die bevorzugte Flexibilität der Elektrode selbst kann mit unterschiedlichen Ausführungen der Elektrode realisiert werden. In einer besonders bevorzugten Ausführungsform besteht die Elektrode aus einem federelastischen Material mit starren Endstücken, wobei die Endstücke zur drehbaren Lagerung wie auch zur elektrischen Kontaktierung dienen können. Hierzu ist wenigstens eins der starren Endstücke elektrisch leitbar und mit der Hochspannungsquelle kontaktierbar ausgebildet.

Das federelastische Material kann eine Spiralfeder sein, sodass die Elektrode aus einer hohlen Spiralfeder gebildet ist.

In anderen Ausführungsformen kann die flexible Ausbildung der Elektrode auch durch massive federelastische Materialien, die elektrisch leitend sind, gebildet sein. Es ist ferner möglich, die Federelastizität durch eine schlauchförmige Hülle der Elektrode zu gewährleisten, die mit einem elektrisch leitenden Material, auch in Form einer Flüssigkeit, gefüllt sein kann. Die Flüssigkeit bildet dabei den leitenden Teil der Elektrode, während die federelastische Hülle leitend sein kann, jedoch nicht muss. Dabei wäre es auch denkbar, die federelastische Hülle als das Dielektrikum auszubilden, in dem sich ein verformbares leitendes Material befindet, mit dem das definierte Hochspannungspotential in einen durch das Dielektrikum definierten Abstand zur zu behandelnden Oberfläche gebracht wird.

In einem bevorzugten Ausführungsbeispiel umgibt das Griffgehäuse die Rolle nahezu komplett und lässt nur eine kleine Öffnung frei, aus der ein nur geringer Umfangsabschnitt der Rolle herausragt. Der herausstehende Umfangsabschnitt kann sich daher über 20 bis 120 Winkelgrade erstrecken.

In einer Variante der Erfindung kann ein Griffgehäuse auch mehrere Rollen lagern, die in Bewegungsrichtung nacheinander über die zu behandelnde Oberfläche geführt werden. Ferner ist es möglich, eine Rolle über ihre axiale Länge aus mehreren Teilrollen aufzubauen.

Die Erfindung soll im Folgenden anhand von Ausführungsbeispielen näher erläutert werden. Es zeigen:
- Figur 1: eine teilweise geschnittene Gesamtdarstellung eines erfindungsgemäßen Plasma-Behandlungsgeräts;
- Figur 2: eine vergrößerte Darstellung einer ersten Ausführungsform einer Rolle mit einer flexiblen Elektrode;
- Figur 3: eine Darstellung des Plasma-Behandlungsgeräts gemäß Figur 1 einer Ansicht der Oberfläche eines Ausführungsbeispiels eines Dielektrikums;
- Figur 4: eine schematische Ansicht eines zweiten Ausführungsbeispiels einer erfindungsgemäß ausgebildeten Rolle;
- Figur 5: eine Schnittdarstellung durch die Rolle gemäß Figur 4.

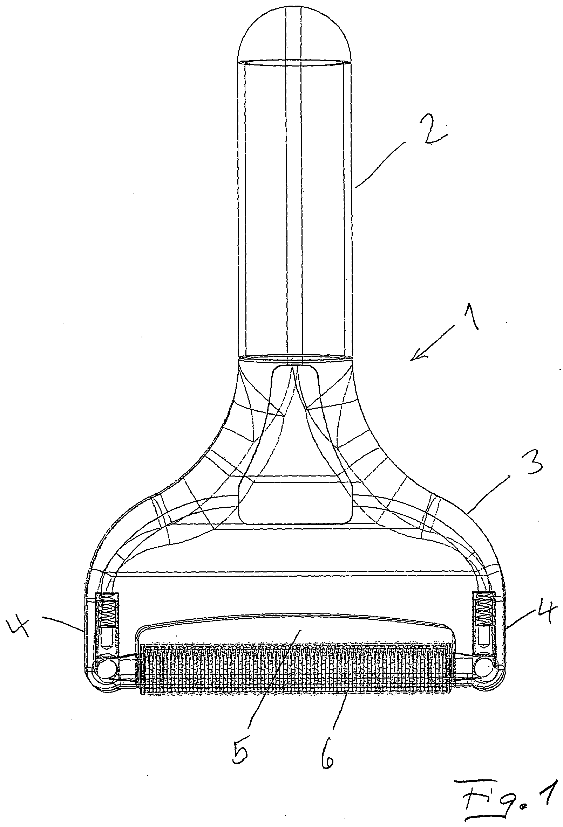

Wie das Ausführungsbeispiel gemäß Figur 1 zeigt, kann das Plasma-Behandlungsgerät ein Griffgehäuse 1 aufweisen, das einen Griffabschnitt 2 und einen Halteabschnitt 3 aufweist. Der Griffabschnitt 2 ist zum Umgreifen mit einer Hand geeignet und kann mit üblichen, den Griff sichernden rutschfesten Oberflächen versehen sein.

Der Halteabschnitt 3 weist zwei vom Griffabschnitt 2 weg weisende parallele Arme 4 auf, zwischen denen sich ein Freiraum 5 befindet. An den Enden der Arme 4 ist eine Elektrodenanordnung in Form einer Rolle 6 drehbar gelagert, wie dies unten näher erläutert wird. Die in den Freiraum 5 eingesetzte Rolle 6 ist durch das Griffgehäuse 1 an ihrer Oberseite und Unterseite nahezu vollständig abgedeckt und ragt nur mit einem kleinen Umfangsabschnitt der Rolle 6 aus dem Griffgehäuse 1 heraus. Dies hat zur Folge, dass das Griffgehäuse 1 mit dem Griffabschnitt 2 im Wesentlichen senkrecht zur zu behandelnden Oberfläche gehalten werden muss, damit ein Umfangsabschnitt der Rolle 6 den gewünschten Kontakt zur (nicht dargestellten) zu behandelnden Oberfläche erhält.

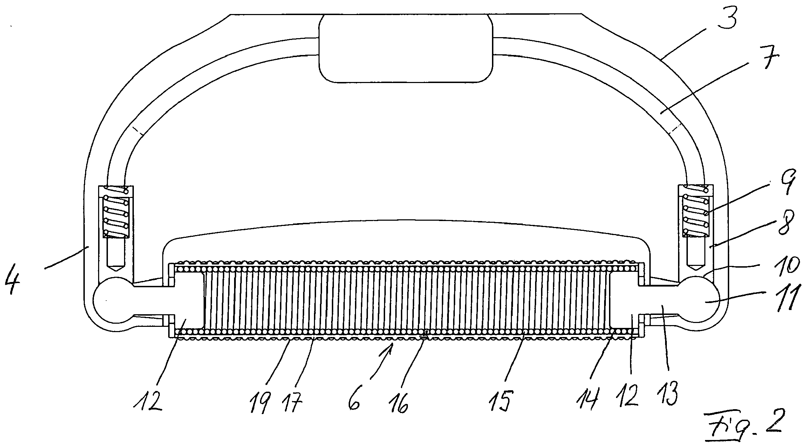

Figur 2 zeigt in einer vergrößerten Schnittdarstellung einige konstruktive Einzelheiten des Halteabschnitts 3 des Griffgehäuses 1 und der Rolle 6 in einer ersten Ausführungsform. Der Halteabschnitt 3 weist zu den beiden Armen 4 hin verlaufende Kanäle 7 auf, in denen Hochspannungsleitungen geführt werden. Diese werden verbunden mit einem metallischen Hohlzylinder 8, der in einer zugehörigen Kammer des Halteabschnitts 3 axial etwas verschiebbar geführt ist. Eine sich im Hohlzylinder 8 abstützende Druckfeder 9 drückt den Hohlzylinder, der einen konkave Stirnfläche 10 aufweist, gegen einen Kugelabschnitt 11 eines metallischen Endstücks 12 der Rolle 6. Das metallische Endstück 12 ist drehfest mit der Rolle 6 verbunden und mit einem zylindrischen Abschnitt 13 in einem entsprechenden Kanal des Halteabschnitts 3 drehbar gelagert. Demgemäß kann der Kugelabschnitt 11 relativ zu der konkaven Stirnfläche 10 rotieren, wobei der elektrische Kontakt durch den Andruck der Druckfeder 9 erhalten bleibt.

Das Endstück 12 ragt mit einem gegenüber dem zylindrischen Abschnitt 3 einen größeren Durchmesser aufweisenden Bolzenabschnitt 14 passend in eine hohlzylindrische Anordnung einer Spiralfeder 15, die an ihren beiden Enden mit einem metallischen Endstück 12 abgeschlossen und drehbar in dem Halteabschnitt 3 des Griffgehäuses 1 gelagert ist. Zwischen den beiden Endstücken 12 ist die Spiralfeder 15 mit aneinander anliegenden Windungen der Spiralfeder ausgebildet. Die Spiralfeder 15 bildet mit den metallischen Endstücken 12 eine längliche, drehbar gelagerte Elektrode 16, die aufgrund der Spiralfeder 15 durch Unebenheiten der zu behandelnden Oberfläche lokal auslenkbar ist und dabei Krümmungen der zu behandelnden Oberfläche beim Abrollen folgen kann.

Die so gebildete Elektrode 16 ist auf ihrer gesamten Mantelfläche von einem schlauchförmigen Dielektrikum 17 umgeben. Das Dielektrikum 17 besteht aus einem flexiblen Material, das die beschriebene Verformung der flexiblen Elek-trode ermöglicht und mitmacht. Das Dielektrikum 17 wird an beiden Stirnenden durch jeweils eine Ringscheibe 18 abgeschlossen, die jeweils eine den Durchtritt des zylindrischen Abschnitts 13 erlaubende Durchgangsöffnung aufweist und an der sich das jeweilige Ende der Spiralfeder 15 abstützen kann.

Das Dielektrikum 17 besteht aus einem weichelastischen Material, vorzugsweise aus TPE und weist an seiner äußeren Oberfläche eine Vielzahl von Erhebungen 19 in Form kleinwulstartiger Noppen auf.

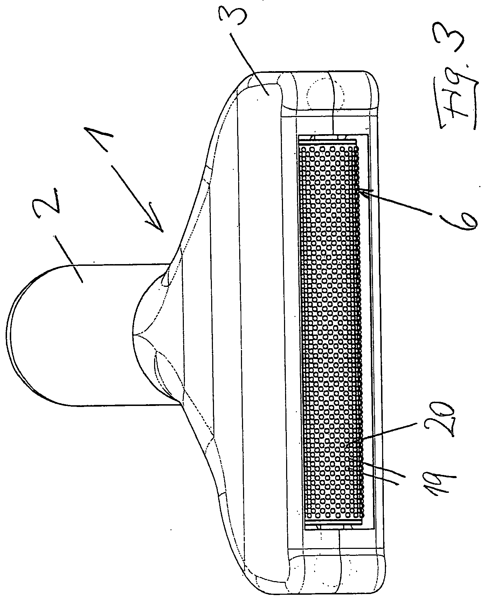

Die Ansicht der Figur 3 verdeutlicht, dass die Erhebungen 19 domartige kleine Noppen sind, die in etwa die Form einer Halbkugel aufweisen, wobei der Radius der Noppen vorzugsweise zwischen 0,2 und 0,5 mm liegen kann, sodass sich ein Noppendurchmesser von 0,4 bis 1,0 mm und eine Höhe der Noppen zwischen 0,2 und 0,5 mm ergibt. Die Erhebungen 19 sind in dem in Figur 3 dargestellten Ausführungsbeispiel axial in Reihen ausgerichtet angeordnet, wobei die Erhebungen 19 benachbarter axialer Linien zueinander versetzt angeordnet sind. Hieraus ergibt sich ein jeweiliger Zwischenraum 20 zwischen zwei Noppen (in beliebiger Richtung) der auch etwa dem Noppendurchmesser entspricht oder etwas größer sein kann. Die Zwischenräume 20 sind miteinander verbunden, sodass sich an der Oberfläche keine durch Erhebungen 19 abgeschlossenen Zellen ausbilden. Auch eine solche Anordnung, beispielsweise durch wabenförmige Erhebungen wäre möglich, wird aber als Ausführungsform nicht bevorzugt.

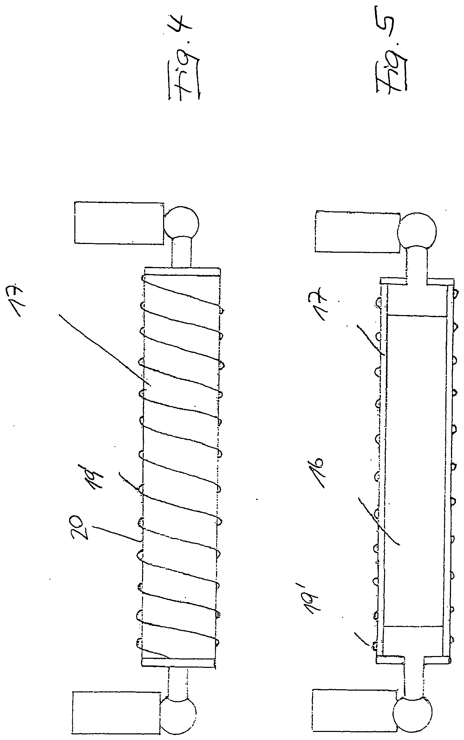

Die in den Figuren 4 und 5 dargestellten Ausführungsform einer Elektrode 16 unterscheidet sich von der Ausführungsform in den Figuren 2 und 3 lediglich dadurch, dass das Dielektrikum 17 Erhebungen 19' in Form eines spiralförmig mit größeren Abständen umlaufenden Drahts gebildet sind, der mit der Oberfläche des Dielektrikums 17 verbunden ist. Die Erhebungen 19' in Form des Drahts können einstückig mit dem Dielektrikum 17 ausgebildet sein oder nachträglich auf die Oberfläche des Dielektrikums 17 aufgebracht sein. Das Material der Erhebung 19' kann beliebig sein, wenn die Erhebung 19' separat von dem Dieelektrikum 17 ausgebildet ist. Die Erhebung 19' kann aus einem leitenden oder vorzugsweise nichtleitenden Material bestehen. Als leitendes Material würde die Erhebung 19' Massepotential annehmen, wenn es mit der zu behandelnden, auf Masse gelegten Oberfläche in Kontakt kommt. Ein Stromfluss von den Erhebungen 19' zur Elektrode 16 wird durch das Dielektrikum 17 sicher verhindert. Es ist erkennbar, dass die Erhebungen 19' auf die Oberfläche eines Dielektrikums 17 nachträglich in einfacher Weise aufgebracht und dort fixiert werden könnte, um so den Abstand zwischen der Oberfläche des Dielektrikums 17 und der zu behandelnden Oberfläche herzustellen, in dem sich ein definierter Luftraum zur Ausbildung des Plasmafelds befindet. Es ist ferner erkennbar, dass beim Abrollen der Rolle 6 auf der zu behandelnden Oberfläche die jeweiligen Kontaktpunkte zwischen der Erhebung 19' und der zu behandelnden Oberfläche wandern, sodass eine gleichmäßige Plasmabehandlung der Oberfläche gewährleistet ist.

Die dargestellten Ausführungsbeispiele sind in keiner Weise beschränkend gemeint. Andere Formen und Muster der Erhebungen 19, 19' sind im Rahmen der Erfindung ohne weiteres realisierbar. Die Erfindung wird durch die beigefügten Ansprüche definiert.

Ferner obliegt es der Dimensionierung nach fachmännischen Gesichtspunkten, welche Höhe der Erhebungen 19, 19' für die jeweilige Behandlungsaufgabe sinnvoll vorgesehen wird. Im Allgemeinen wird jedoch eine Erhebung zwischen 0,2 und 1,5 mm zutreffend sein. Die von den Erhebungen 19, 19' eingenommene Fläche sollte gegenüber der Fläche der Zwischenräume 20 einen Anteil von 1 bis 15 % einnehmen. Wesentlich ist dabei, dass die Erhebungen 19, 19' immer zu punktförmigen oder kleinflächigen Kontakten mit der zu behandelnden Oberfläche führen und größere zusammenhängende Kontaktflächen vermieden werden.

## Patentansprüche

1. Plasma-Behandlungsgerät zur Behandlung einer Oberfläche mit einem dielektrisch behinderten Plasmafeld, das zwischen einer mit Hochspannung versorgten Elektrode (16) und der Oberfläche erzeugt wird, wobei die Elektrode (16) mit einem die Elektrode (16) umgebenden Dielektrikum (17) eine drehbar in einem Griffgehäuse (1) gelagerte Rolle (6) bildet, die auf der Oberfläche abrollbar ist, **dadurch gekennzeichnet, dass** die Rolle (6) an Unebenheiten der Oberfläche flexibel anpassbar ausgebildet ist und eine Abrollfläche mit Erhöhungen (19, 19') aufweist, zwischen denen sich das Plasmafeld ausbildende Zwischenräume (20) befinden.

2. Plasma-Behandlungsgerät nach Anspruch 1, **dadurch gekennzeichnet, dass** die Zwischenräume (20) miteinander verbunden sind.

3. Plasma-Behandlungsgerät nach Anspruch 1 oder 2, **dadurch gekennzeichnet, dass** das Dielektrikum (17) aus einem weichelastischen Kunststoff besteht.

4. Plasma-Behandlungsgerät nach Anspruch 3, **dadurch gekennzeichnet, dass** der weichelastische Kunststoff eine Härte Shore (A) zwischen 30 und 60 aufweist.

5. Plasma-Behandlungsgerät nach einem der Ansprüche 1 bis 4, **dadurch gekennzeichnet, dass** die Rolle (6) mit ihren beiden Enden drehbar in dem Griffgehäuse (1) gelagert ist.

6. Plasma-Behandlungsgerät nach einem der Ansprüche 1 bis 5, **dadurch gekennzeichnet, dass** die Elektrode (16) flexibel ausgebildet ist.

7. Plasma-Behandlungsgerät nach Anspruch 6, **dadurch gekennzeichnet, dass** die Elektrode (16) aus einem federelastischen Material mit starren Endstücken (12) gebildet ist.

8. Plasma-Behandlungsgerät nach Anspruch 7, **dadurch gekennzeichnet, dass** federelastische Material eine Spiralfeder (15) ist.

9. Plasma-Behandlungsgerät nach Anspruch 7 oder 8, **dadurch gekennzeichnet, dass** wenigstens eins der starren Endstücke (12) elektrisch leitend und mit einer Hochspannungsquelle kontaktierbar ausgebildet ist.

## Claims

1. A plasma treatment device for treating a surface with a dielectric barrier plasma field, which is generated between an electrode (16) supplied with high voltage and the surface, the electrode (16) with a dielectric (17) enclosing the electrode (16) forming a roller (6), mounted rotatably in a handle housing (1), which can be rolled on the surface **characterized in that** the roller (6) is configured to be flexibly adaptable to irregularities of the surface and has a rolling surface with elevations (19, 19'), between which interspaces (20) for forming the plasma field are located.

2. The plasma treatment device as claimed in claim 1, **characterized in that** the interspaces (20) are connected to one another.

3. The plasma treatment device as claimed in claim 1 or 2, **characterized in that** the dielectric (17) consists of a highly flexible plastic.

4. The plasma treatment device as claimed in claim 3, **characterized in that** the highly flexible plastic has a Shore (A) hardness of between 30 and 60.

5. The plasma treatment device as claimed in one of claims 1 to 4, **characterized in that** the roller (6) is mounted rotatably with its two ends in the handle housing (1).

6. The plasma treatment device as claimed in one of claims 1 to 5, **characterized in that** the electrode (16) is configured to be flexible.

7. The plasma treatment device as claimed in claim 6, **characterized in that** the electrode (16) is formed from a resilient material with rigid endpieces (12).

8. The plasma treatment device as claimed in claim 7, **characterized in that** the resilient material is a coil spring (15).

9. The plasma treatment device as claimed in claim 7 or 8, **characterized in that** at least one of the rigid endpieces (12) is configured to be electrically conductive and contactable by a high-voltage source.

## Revendications

1. Dispositif de traitement au plasma pour traiter une surface par un champ de plasma à barrière diélectrique qui est généré entre une électrode (16) alimentée sous haute tension et la surface, l'électrode (16) formant, conjointement avec un diélectrique (17) entourant l'électrode (16), un rouleau (6) monté rotatif dans un boîtier à poignée (1) susceptible de rouler sur la surface, **caractérisé en ce que** le rouleau (6) est réalisé avec faculté d'ajustement flexible à des irrégularités de la surface et comprend une surface de roulement pourvue de surélévations (19, 19') entre lesquelles se trouvent des interstices (20) réalisant le champ de plasma.

2. Dispositif de traitement au plasma selon la revendication 1, **caractérisé en ce que** les interstices (20) sont reliés entre eux.

3. Dispositif de traitement au plasma selon la revendication 1 ou 2, **caractérisé en ce que** le diélectrique (17) est constitué en une matière plastique élastique et souple.

4. Dispositif de traitement au plasma selon la revendication 3, **caractérisé en ce que** la matière plastique élastique et souple présente une dureté Shore (A) comprise entre 30 et 60.

5. Dispositif de traitement au plasma selon l'une des revendications 1 à 4, **caractérisé en ce que** le rouleau (6) a ses deux extrémités montées rotatives dans le boîtier à poignée (1).

6. Dispositif de traitement au plasma selon l'une des revendications 1 à 5, **caractérisé en ce que** l'électrode (16) est réalisée de façon flexible.

7. Dispositif de traitement au plasma selon la revendication 6, **caractérisé en ce que** l'électrode (16) est formée en un matériau présentant l'élasticité d'un ressort et pourvue d'embouts d'extrémité rigides (12).

8. Dispositif de traitement au plasma selon la revendication 7, **caractérisé en ce que** le matériau présentant l'élasticité d'un ressort est un ressort spiralé (15).

9. Dispositif de traitement au plasma selon la revendication 7 ou 8, **caractérisé en ce que** l'un au moins des embouts d'extrémité rigides (12) est électriquement conducteur et réalisé de manière à pouvoir venir en contact avec une source de haute tension.
